# EUROPEAN PATENT SPECIFICATION

(11) **EP 2 410 930 B1**
(45) Date of publication and mention of the grant of the patent: **26.02.2014**
(21) Application number: 10710702.1
(22) Date of filing: 10.03.2010
(51) Int. Cl.: A61B 17/58, A61B 17/70, A61B 17/86, A61F 2/30

(54) **IMPROVED ORTHOPEDIC IMPLANT**
VERBESSERTES ORTHOPÄDISCHES IMPLANTAT
IMPLANT ORTHOPÉDIQUE AMÉLIORÉ

(30) Priority: 24.03.2009 US 162697 P; 11.09.2009 US 557577
(43) Date of publication of application: 01.02.2012
(73) Proprietor: X-spine Systems, Inc., Miamisburg, OH 45342 (US)
(72) Inventor: KIRSCHMAN, David, Louis, Dayton OH 45429 (US); MANNAVA, Seetha, Ramaiah, Cincinnati OH 45421 (US); VASUDEVAN, Vijay, K., Cincinnati OH 45242 (US)
(74) Representative: Rüfenacht, Philipp Michael
(86) International application number: PCT/US2010/026752
(87) International publication number: WO 2010/111033

(56) References cited:
- JP-A- 2006 322 446
- US-A1- 2001 014 807
- US-A1- 2004 148 033
- US-A1- 2006 009 857
- US-A1- 2007 186 990
- US-A1- 2008 221 681
- US-B1- 6 911 100

## Description

### CROSS-REFERENCE TO RELATED APPLICATION

The present application claims priority to provisional U.S. Application Serial No. 61/162,697 filed March 24, 2009, and to U.S. Application Serial No. 12/557,577 filed September 11, 2009, and published as US 2010/0249926 to which Applicant claims the benefit of the earlier filing date.

### BACKGROUND OF THE INVENTION

### 1. Field of the Invention

This invention relates to orthopedic implants and, more particularly, to improved orthopedic implants.

### 2. Description of the Related Art

Implanted instrumentation such as pedicle screw system is a mainstay of spinal fixation procedures in the thoracic and lumbar spine. These implants are used to help join vertebrae together and restore stability. Traditionally, spinal implant products have been rigid constructs designed to hold the spine immovably in place. Rigid fixation allows for an irreversible biological fusion of the vertebrae together. The natural mechanical stability of the pedicles makes them an optimal site for attaching posterior fixation devices to achieve immediate spinal stability.

Traditional pedicle screw fixation systems are multi-component devices. Typical implant consists of plates, rods, and screws. Pedicle screws are designed and sized to anchor in the bone of the pedicles. The screw thread extends the entire length of the pedicle, and is terminated posteriorly by a screw head that is designed to mate with rigid rods that are longitudinally interconnected and anchored to adjacent vertebrae using additional hooks or pedicle screws. The rods provide mechanical stability between adjacent vertebrae while the screws provide anchoring.

The portion of the pedicle screw system which determines its rigidity is the rod. The rod is typically manufactured from titanium alloy and has a diameter of 5.5 to 6.35 mm. Stainless steel titanium is used because of its good mechanical properties and lack of rejection by the body. This rod needs to tolerate the loads of the spinal column. Such loads are tension, torsion, and compression over multiple cycles.

The current industry standard is that a rigid-type rod construct must tolerate 5 million load cycles without failure. The rods are subject to fatigue failure at high cycle/high load conditions. The typical failure mode is a fatigue crack of the rod at either a midpoint of the rod shaft or at a connection point with the screw or rod connector. It is mandatory to conduct laboratory mechanical tests to determine the static and dynamic strength capability of typical constructs according to ASTM test procedures. The compressive fatigue run-out load of the rods is approximately 50% of the static load. If the loads exceed this value after they are implanted in the patient, the rods will fail and have to be removed surgically. In implanted rigid devices, fatigue failure occurs at a rate of approximately 0.5 - 1%. Such failures typically occur in patients where there are an abnormally high loads placed upon the implant. This can occur in situations of patient obesity, failure of the fusion to heal properly, or high levels of patient activity during the post-operative period. Thus, it is desirable to increase fatigue strength of these rods as high as possible.

Although commonly used, rigid fusion of the spine has several important drawbacks. The reduction in spinal motion which occurs in such procedures reduces the amount of range of motion in the patient. This has negative repercussions in quality of life and may hamper the patient's ability to return to work. Additionally, when one segment of the spine is rigidly held together, the mechanical loads are then transferred to adjacent, untreated segments. This is known as the "transitional segment phenomenon" which can result in the need for subsequent surgeries at additional spinal levels.

Dynamic stabilization is a newer technology developed to provide stability to the spinal segments without the need for rigid fusion, a procedure which alters the spine's biomechanics and may lead to degeneration of adjacent vertebral segments. Dynamic stabilization devices are designed to support the spine from the posterior (rearward) side, sharing load with the spine, and leaving the spinal anatomy relatively intact without fusing the vertebrae. The key principles of dynamic stabilization are based on the premise that the ability to control abnormal motions of spinal segments and provide a more natural load transmission will eliminate pain and prevent further degeneration of adjacent vertebral segments. Additionally, dynamic stabilization procedures have the advantage of reducing or eliminating the need for bone grafting, resulting in reduced treatment costs and surgery time. A disk augmentation system utilizes adjustable elastomers as well as metallic technology to achieve dynamic fixation of the lumbar spine; the next evolution in the surgical treatment of degenerative disk disease.

There are advantages to improving the performance of implants and making them stronger and more fatigue-resistant. A typical approach is to increase the size of the implant (e.g., increasing a circumference of a rod implant), but this has drawbacks. For example, increasing a size of the implant can make it difficult to implant Increasing the size of an implant also results in an increase in cost of the implant.

US 2006/009857 discloses implants having a hardened surface as well as methods to harden the surface of an implant. A portion of an articulation surface of a prosthesis is hardened by treatment with high energy laser electromagnetic radiation. For example, a curved spherical surface of a femoral head or a portion of a coupling surface of a male portion of a morse taper are hardened by laser shock peening.

US 2008/0221681 describes a method to introduce a residual compressive stress into a body portion of an implantable device. The compressive stress is introduced to maintain fatigue resistance properties of the implantable device or a portion thereof. As an example, an implantable device may be shot peened to impart compressive stress. The work-hardened surface may be subjected to additional texturing treatment, such as grit blasting.

JP 2006-322446 relates to improvements of internal pressure fatigue strength of common rails for fuel injection systems. Laser peening is applied to an opening peripheral part of a branch point of a common rail. The average application time of the laser pulses to the same point in the opening is set from 2 to 100, thus setting the uniaxial tensile strength of the steel material to 30 to 120% of said predefined zone.

US 2001/0014807 discloses an implant system comprising a plate, screws and expandable/contractible rings. To increase the coefficient of friction between the head of the screws and the rings, a number of textured surfaces may be used which includes a ball peening process. Either the outer surface of the screw head and/or the inner surface of the ring may be textured by ball peening. The texturation inhibits movement of the screw in relation to the plate.

US 2004/0148033 describes prosthetic devices with a hardened articulating surface to increase the longevity and to decrease possible wear debris. The hardening may be performed on the stock material used to produce the prosthetic devices or on the rough formed prosthetic devices. Different hardening methods may be used, such as shot peening, roller hardening or laser shock peening. Such as to induce a desired hardness on the entire stock material, an intense working such as roller working is preferred.

US 6,911,100 describes a method for producing predetermined internal stresses in a prosthetic device. The Internal stresses may be used to increase the strength of smaller prosthetic devices, Residual stresses required in the prosthetic device to withstand anticipated loads are first determined and then created in the prosthetic device by using an appropriate cold working method, such as shot peening or laser shock peening.

US 2007/186990 relates to an elongated member for use in orthopaedic procedures for supporting or correcting tissue. The elongated member includes four sections, three of which are substantially cylindrical. One section has two substantially planar surfaces that are opposed to each other, This plate section may be used to connect the elongated member to a vertebra via screws inserted through openings provided in the section having planar surfaces.

What is needed, therefore, is an improved implant that can be used in a dynamic stabilization procedure.

### SUMMARY OF THE INVENTION

An object of the invention is to provide a dynamic stabilization device that has flexibility to accommodate the motions of the spinal segments coupled with high fatigue strength to meet high fatigue loads and longer implant durations.

What is needed, therefore, is an improved implant that can be used in a dynamic stabilization procedure and that has improved performance characteristics and a system and method for making such implant and that overcomes one or more of the drawbacks in the past.

### SUMMARY OF THE INVENTION

The present invention relates to an orthopaedic implant as claimed hereafter. Preferred embodiments of the invention are set forth in the dependent claims.

An object of the invention is to provide a dynamic stabilization device that has flexibility to accommodate the motions of the spinal segments coupled with high fatigue strength to meet high fatigue loads and longer implant durations.

Another object of the invention is to provide an implant that accomplishes such flexibility by designing, shaping or reshaping the implant geometry, for example, from a circular cross section to a rectangular cross section or to have planar or treatment areas at selected locations to optimize compression and densification in those areas.

Another object of this technology is to provide an implant that will augment, rather than replace, regenerating spinal motion elements, including disks and facet joints.

Another object of the invention is to provide an implant that is capable of accommodating high fatigue loads endemic to dynamic devices by using laser shock peening, ultrasonic or other peening, burnishing and compression techniques to improve the performance of the implant in a disk or other augmentation system.

Still another object of the invention is to provide an implant having at least one predetermined zone or processing zone that has been designed to optimize compression, such as by compression ultrasonic or laser shock peening.

Yet another object of the invention is to provide an implant having biomechanical stress concentration at areas of fatigue, wherein such areas are provided in a predetermined pattern, such as linear, arcuate, overlapping, spherical, helical or interrupted.

Still another object of the invention is to provide an Implant having compressed or densified areas at predetermined areas in the implant, such as a point or area that is equidistant between the contact points where the implant is mounted to a skeletal structure.

Another object of the invention is to improve fatigue strength, which will have at least the following advantages:
1. Increased cycles to fatigue fallure will enable the use of the spinal implant construct for longer periods thus usable in fusion as well as in fusionless procedures.
2. Increased fatigue load ratio will enable the spinal construct to withstand the abnormal high fatigue loads that may occur during pseuarthrosis (fusion failure).
3. Decrease the rod diameter to reduce fixation profile, thereby increasing the use of the constructs at various anatomical locations.

In one aspect, one embodiment comprises an orthopedic implant comprising an implant body, a first portion of the implant body have a first density, and a second portion of the implant body has a second density, wherein the first portion is compressed so that the first density is higher than the second density associated with the second portion, the first portion comprising a biomechanical stress concentration or density that is higher than a biomechanical stress concentration or density in the second portion when the orthopedic implant is subject to biomechanical forces after being situated on a skeletal structure.

Method for processing an orthopedic implant which does not form part of the present invention comprises the steps of providing an implant body, determining areas of stress in the orthopedic implant during use in a patient, using the areas of stress to determine at least one predetermined zone in the implant body to facilitate or substantially optimize compressive stressing of the at least one predetermined zone, and compressive stress processing the at least one predetermined zone of the implant body such that after the compressive stress processing step, the at least one predetermined zone comprises a biomechanical stress concentration or first density at the at least one predetermined zone that is generally higher than a biomechanical stress concentration or second density in other areas of the implant body when the orthopedic implant is subject to biomechanical forces after being situated on a skeletal structure.

Preferred embodiments of this method for processing an orthopedic implant which does not form part of the present invention comprises the following features either alone or in combination:
- Said compressive stress processing step comprises the step of peening said at least one predetermined zone by ultrasonic or laser peening.
- Said at least one predetermined zone comprises generally planar or flat areas in said orthopedic Implant.
- Said method further comprises the step of sterilizing said implant body using at least one of irradiation, heat or chemically.
- Said method not belonging to the present invention comprises the step of processing said implant body after said compressive stress processing step in order to correct dimensional intolerances or to configure said implant body to a desired shape or dimension.
- Said implant body is a plate, cage screw or rod.
- Said orthopedic implant is a screw and said at least one predetermined zone is a shank of said screw.
- Said orthopedic implant is a plate and said at least one predetermined zone comprise areas around screw openings in said plate.
- Said orthopedic implant is a rod and said at least one predetermined zone is a generally planar surface along a length of said rod.
- Said compressive stress processing step further comprises the step of laser or ultrasonically peening said at least one predetermined zone in a predetermined pattern.
- Said compressive stress processing step further comprises the steps of:
   - laser shock peening said at least one predetermined zone in a predetermined pattern using a laser;
   - causing relative movement of said implant body with respect to said laser to create said predetermined pattern.
- Said predetermined pattern is rectangular, circular, elliptical, polyaxial, helical, linear, curved or overlapping, or spiral.
- Said predetermined pattern is discontinuous or interrupted along at least a length or a width of said orthopedic implant.
- Said providing step comprises the step of providing an implant body that is elongated and that comprises a plurality of peripherally-spaced lobes, a portion of at least one of said plurality of peripherally-spaced lobes extending longitudinally along said implant body and adapted to define said at least one predetermined zone.
- Said compressive stress processing step comprises the step of peening or burnishing said at least one of said plurality of peripherally-spaced lobes to define said at least one predetermined zone.
- Said plurality of peripherally-spaced lobes comprises a first lobe and a generally opposing second lobe, a portion of each of said first and second lobes being densified by laser peening to provide a plurality of predetermined zones.
- Each of said plurality of peripherally-spaced lobes comprise tapered sides.
- Said compressive stress processing step comprises the step of compressive stress processing selective ones of said plurality of peripherally-spaced lobes to define a plurality of predetermined zones having different densities compared to at least one other of said plurality of peripherally-spaced lobes that are not compressive stress processed.
- Selective ones of said plurality of peripherally-spaced lobes are compressive stress processed to comprise a thickness in cross section that is less than a second thickness of said at least one other of said plurality of peripherally-spaced lobes that were not compressive stress processed.
- Said method which does not belong to the present invention further comprises the steps of:
   - identifying surface distortions, modifications or further processing required on said implant body resulting from said compressive stress processing step;
   - processing said implant body further to remove or adjust for said surface distortions, modifications or to perform said further processing.
- Said method which does not form a part of the present invention further comprises the step of polishing said orthopedic implant after said compressive stress processing step.
- Said at least one predetermined zone comprises an equidistant area generally equidistant between two fixation points when said orthopedic implant is mounted onto a skeletal structure, said compressive stress processing step comprising the step of compressive stress processing said equidistant area.
- Said method which does not form a part of the present invention comprises the step of adapting said implant body to comprise a plurality of dynamic flexion and load characteristics.
- Said method which does not belong to the present invention comprises the step of adapting said implant body to comprise a plurality of pairs of generally opposing surfaces that comprise said multiple dynamic flexion and load characteristics.
- Said plurality of pairs of generally opposing surfaces are laser shock peened.
- Said method comprises the step of applying a biocompatibte ablation coating to at least a portion of the implant prior to said laser shock peening step.
- Said method which does not form a part of the present invention further comprises the step of removing said biocompatible ablation coating from any areas on said implant where said biocompatible ablation coating remains after said laser shock peening step.

A system for making an implant, said system not forming a part of the present inventioncomprises a holder for holding the implant, a design station for determining areas of stress in the implant during use in a patient and for creating a predetermined design including at least one predetermined zone in the implant to facilitate or substantially optimize compression of the at least one predetermined zone, a processing station for processing the implant at the at least one predetermined zone to facilitate or substantially optimize the compression of the at least one predetermined zone in response to the predetermined design, and a compression station for compressing the at least one predetermined zone of the implant.

Preferred embodiments of this system which does not form a part of the present invention for making an implant comprise the following features either alone or in combination:
- Said compression station comprises at least one peener for peening said at least one predetermined zone by ultrasonic or laser peening.
- Said at least one peener comprises at least one laser peener for laser shock peening said at least one predetermined zone in a predetermined pattern using a laser.
- Said predetermined pattern is discontinuous or interrupted along at least a length or a width of said implant.
- Said compression station comprises a controller coupled to said at least one laser peener for controlling a pulse width, laser energy or laser spot size of said laser to create a predetermined pattern.
- Said predetermined pattern is rectangular, circular, elliptical, polyaxial, linear, overlapping spiral or helical.
- Said compression station further comprises at least one tool for causing relative movement of said implant with respect to said laser peener to create said predetermined pattern at said at least one predetermined zone.
- Said at least one predetermined zone comprises generally planar or generally flat areas.
- Said predetermined design comprises generally flat or generally planar areas.
- Said system further comprises a sterilizing station for sterilizing said implant after said at least one predetermined zone has been compressed.
- Said sterilizing station sterilizes by irradiation, thermally or chemically.
- Said system further comprises a post-compression processing station for processing said implant after said implant is treated at said compressive station in order to correct dimensional intolerances or to configure said implant to a desired shape or dimension.
- Said implant is a plate, cage, screw or rod.
- Said implant is a screw and said at least one predetermined zone is a shank of said screw.
- Said at least one predetermined zone comprises an area generally equidistant between two fixation points when said implant is mounted onto a skeletal structure.
- Said implant is a plate and said at least one predetermined zone comprise areas around screw openings in said plate.
- Said implant is a rod and said at least one predetermined zone is a generally planar surface along a length of said rod.
- Said rod comprises a plurality of lobes, said at least one predetermined zone being at least a portion of at least one of said plurality of lobes.
- Said compression station compressively stresses said at least one predetermined zone in a predetermined pattern.
- Said predetermined pattern is spiral or helical.
- Said system which does not form a part of the present invention further comprises:
   - a finishing station for identifying surface distortions, modifications or further processing required on said implant resulting from said processing said implant at said compression station;
   - said finishing station further comprising a finisher for processing said implant further to remove or adjust for said surface distortions, modifications or to perform said further processing.
- Said system which does not form a part of the present invention further comprises the step of a polishing station for polishing said implant after compressing said at least one predetermined zone at said compression station.
- The implant comprises a biocompatible ablation tape or coating on at least a portion of an outer surface of said implant prior to laser peening.
- The system which does not belong to the present invention comprises a station for removing said biocampatible ablation tape or coating from said at least a portion of said outer surface of said implant that remains after said laser shock peening.
- Said implant body comprises a plurality of dynamic flexion and load characteristics.
- Said implant body comprises a plurality of pairs of generally opposing surfaces that comprise said multiple dynamic flexion and load characteristics.

These and other objects and advantages of the invention, either alone or in combination, will be apparent from the following description, the accompanying drawings and the appended claims.

### BRIEF DESCRIPTION OF THE DRAWINGS

Fig. 1 is a graph illustrating a fatigue strength enhancement or implant process which does not form a part of the present invention:
Fig. 1A is a graph illustrating flexibility, load capacity and fatigue strength of an implant according to an embodiment of the invention;
Fig. 2 is an illustration of a prior art laser shock peening process;
Figs. 3A - 3B are comparisons of residual stress versus depth profiles of prior art airfoils;
Fig. 4 is another not forming part of the invention;
Fig. 5 is a schematic of a process for identifying processing zones which does not belong to the present invention;
Fig. 6 is a schematic showing one illustrative process for correcting dimensional distortions or modifications which is not a part of the present invention;
Figs, 7A - 7B illustrate a which is not a part of the invention;
Fig. 7C - 7D illustrate an exemplary implant, such as a rod, processed in accordance with the system and processes not forming a part of the present invention shown in Figs. 4 - 6;
Fig. 8 is an illustrative implant processed in accordance with a process not forming a part of the present invention;
Fig. 9 is another illustrative implant in the form of a rod processed in accordance with a process not forming a part of the present invention;
Fig. 10 is a another illustrative implant in the form of a polyaxial screw which does not form part of the present invention;
Fig. 11 is a fragmentary view of an illustrative implant not belonging to the present invention in the form of a rod showing overlapping compression areas taken along the line 11 - 11 in Fig. 7C; and
Fig. 12 is a view taken along the line 12 - 12 in Fig. 7C;
Figs. 13A-13C are various views of the rod shown in Fig. 7C and biomechanical stress concentrations along a length of the rod, illustrating various embodiments with the rod being processed to have one generally planar surface (Fig. 13B) or opposed planar surfaces (Fig. 13C);
Figs. 14A and 14B illustrate an implant or rod according to the present invention comprising peripherally-spaced lobes, at least one or a plurality of which have different density resulting from processing and also illustrating different lobe thicknesses;
Figs. 15 and 16 illustrate another embodiment not forming part of the present invention illustrating the laser shock peening on an implant, such as a rod, having a cylindrical or continuous outer radius
Fig. 17 is a view showing various stress profile illustrations for an inventive rod of the type shown in Figs. 14A-14B;
Fig. 18 is a graph illustrating various features of the multiple dynamic flexible rod according to the present invention as shown in Fig. 14A, illustrating a single rod having multiple compression load and flex load characteristics; and
Fig. 19 is a graph illustrating residual stress of an implant versus depth and a theoretical comparison of the LSP-treated implant to a conventional shot peened implant.

### DETAILED DESCRIPTION OF ILLUSTRATIVE EMBODIMENTS

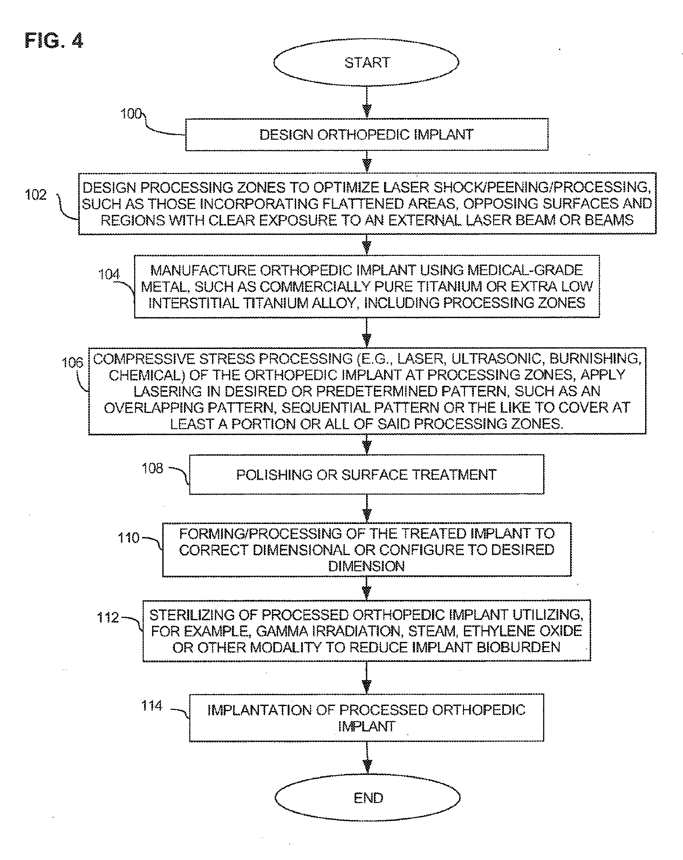

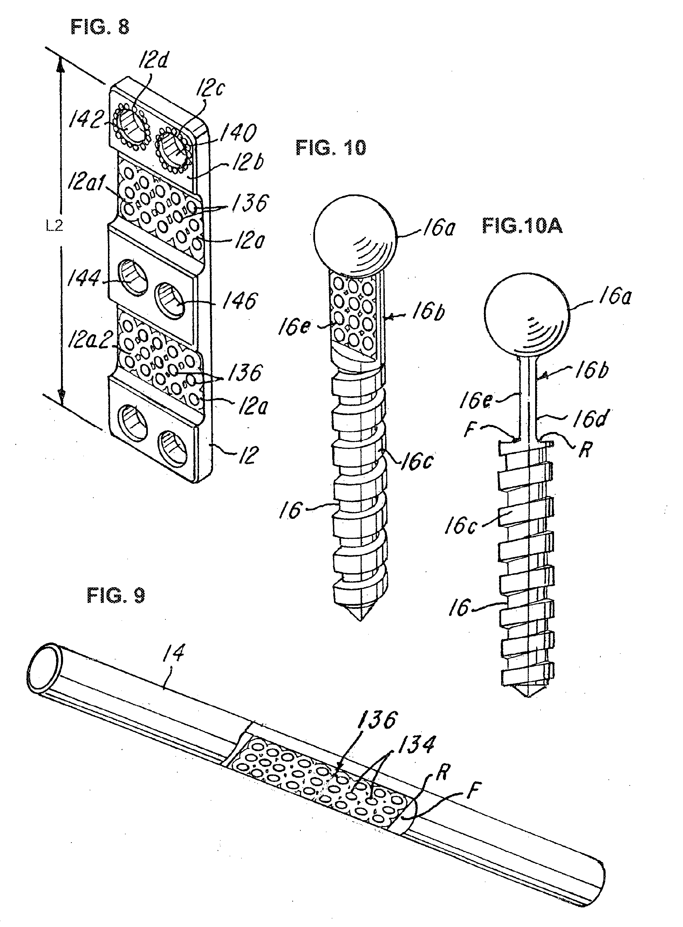

Referring now to Figs. 4-15, an implant, system and method for making an implant, such as an orthopedic implant plate 12 (Fig. 8), rod 14 (Fig. 9) and screw 16 (Fig 10), all not forming a part of the present invention, are shown. For purposes of illustration, an implant in the form of the rod 14 will be described, with it understood that the implant could be any implant, such as those mentioned herein, capable or adapted to be processed and made in accordance with a system and a method described herein but not forming a part of the present invention. One illustrative system, methodology or process for making a dynamic, flexible medical or orthopedic implant, such as the spinal fixation rod 14, disk augmentation system, plate 12, polyaxial screw 16 and the like, not belonging to the present invention will now be described relative to Figs. 4 - 11.

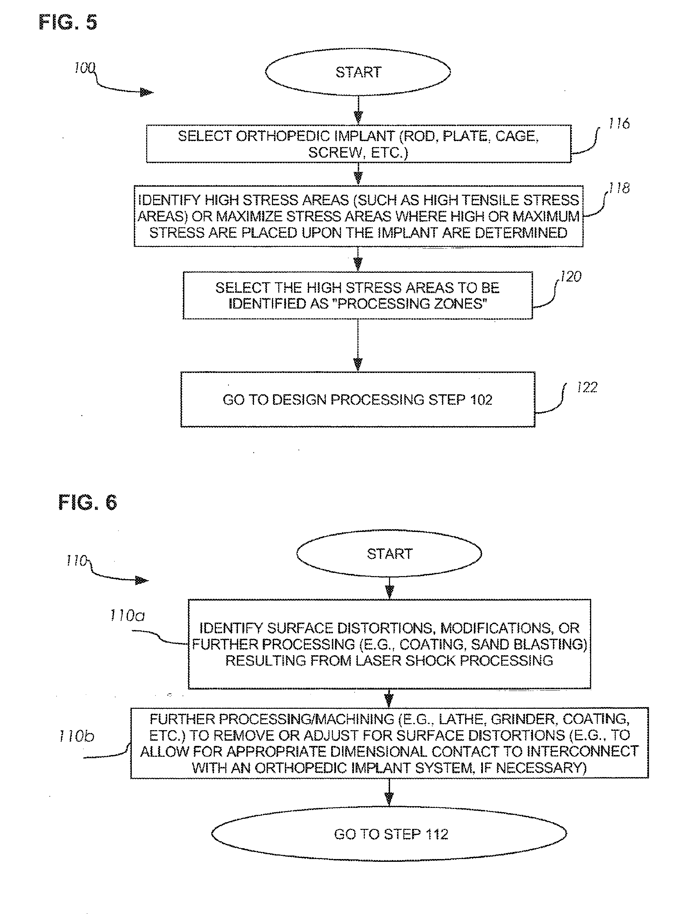

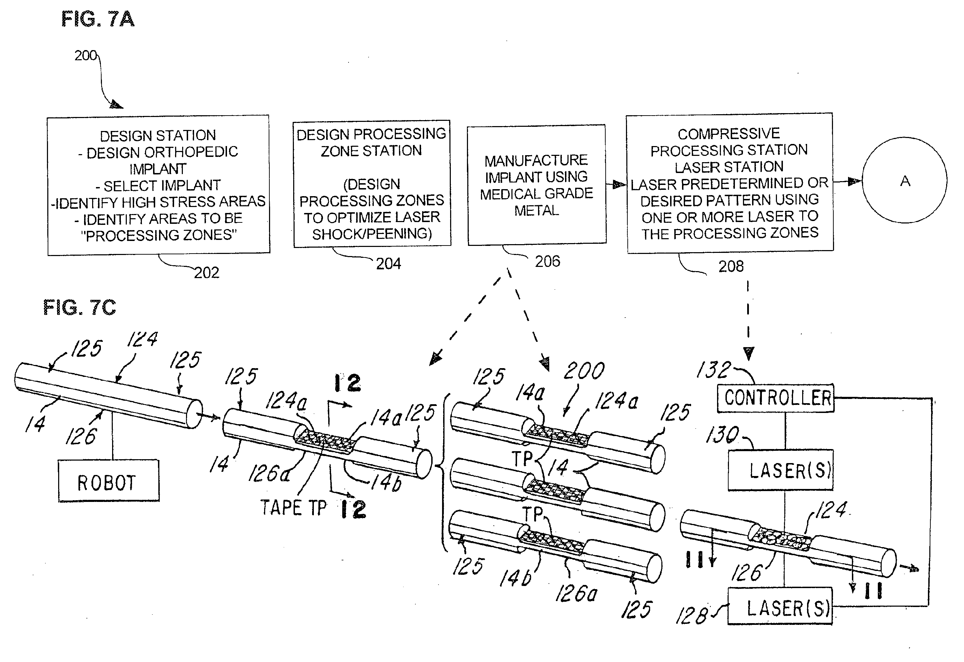

The system 200 (Figs. 7A - 7B) and a related process or procedure (Figs. 4 - 6), both of which do not form a part of the present invention begins at block 100 (Fig. 4) wherein an orthopedic implant, such as the orthopedic implant plate 12 (Fig. 8), rod 14 (Fig. 9) or polyaxial screw 16 (Fig. 10), is selected and designed or redesigned. One illustrative procedure for designing the orthopedic implant is shown in Fig. 5 wherein the design procedure begins by selecting the orthopedic implant to be manufactured (block 116). This occurs at a design station 202 (Fig. 7A) in the system 200. The areas of stress in the implant during use in a patient are determined (block 118) at station 202. The high stress areas are maximum areas where the highest maximum stresses are placed on the implant during use.

At station 204 (Fig. 7A), a predetermined design of the implant is created. The predetermined design includes at least one or a plurality of predetermined zones or processing zones. The at least one or a plurality of predetermined zones or processing zones will be used to adapt or configure the implant to facilitate or substantially optimize the compression of the implant at the at least one or a plurality of predetermined zones or processing zones. At block 118 (Fig. 5) and station 202 (Fig. 7A), at least one, a plurality or all of the high stress areas or zones are determined as mentioned. In this illustration, the identified areas, such as surfaces, areas or zones 124 (Fig. 7C) and 126 in rod 14, are labeled or identified as the at least one "processing zones" or predetermined zones at station 202 and processing zones in the implant are designed at station 204 in response thereto.

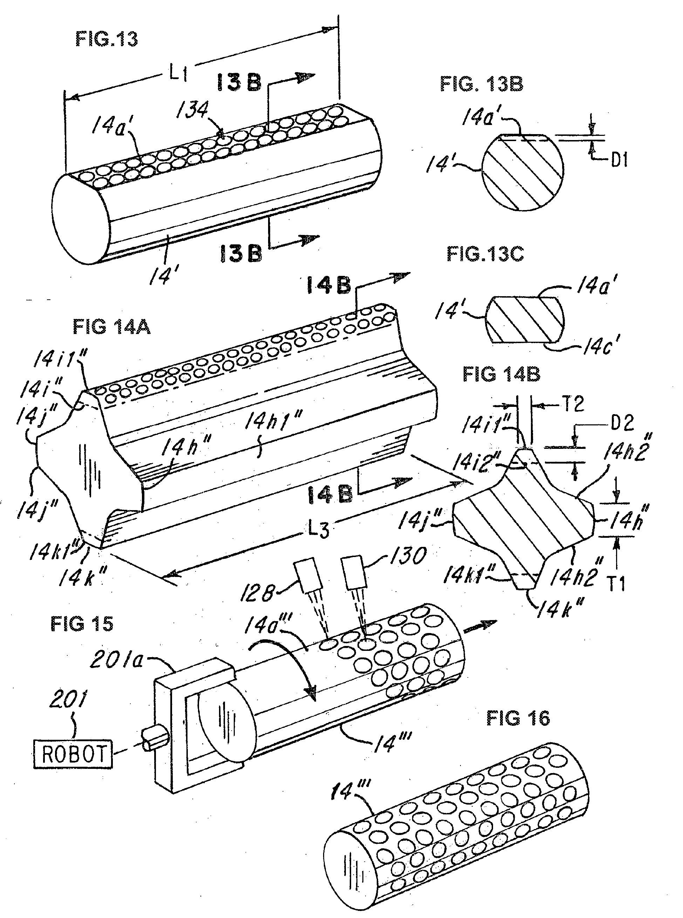

The component or implant (e.g., rod 14) will be fixed to an indexing tool or other means for indexing the implant. One means for indexing is by use of a robot 201 (Figs. 7A and 15) having a robotic arm or holder 201 a (Fig. 15). The robot 201 is coupled to and under control of a controller 132 that is programmed to provide relative movement between the component and the compressor, such as at least one ultrasonic or laser peener. Other conventional means for indexing the implant may be used, such as a rigidly fixture or mechanical indexer. For ease of illustration, the robot 201 is only shown at the upstream end of the process and system 200 in Fig. 7C.

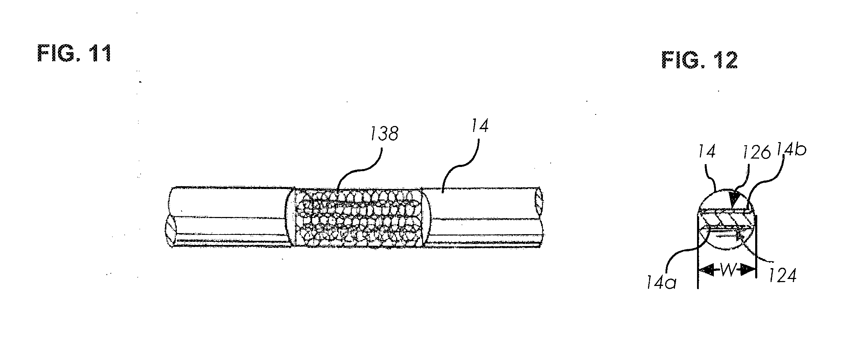

It should be understood that if the implant is compression or processed using laser peening as described later herein, the implant, such as the rod 14 in the illustration in Fig. 7C, is treated with a bio-compatible ablation coating, medium or bid-compatible tape, identified as tape TP in Figs. 7C and 12. This tape TP is applied to at least a portion of implant, such as those areas where the laser peening process described herein is applied, or the tape TP or ablation medium could be applied to the entire implant. For ease of illustration, the rod 14 is shown with biocompatible tape or coating TP applied only to the surfaces 14a and 14b where the rod 14 will be laser peened, but again, it should be understood that the entire surface of the rod 14 may be provided with the ablation medium or tape at any time prior to the implant being processed at the station 208 described later herein, such as after the rod 14 is machined as provided herein. One feature of tape TP or ablation medium being described is that it is biocompatible and conforms with and/or passes ISO 10993. During laser processing as described herein, the tape TP and ablation medium cooperates with the laser beam and a confinement medium, such as water, to create plasma-induced vapor pressure which causes shock waves into the implant, thereby resulting in compression and densification at the area of compression.

As mentioned, the rod 14 is selected (block 116 in Fig. 5) and high stress areas identified (block 118 in Fig. 5, station 202 in Fig. 7A). At block 118 and station 204, the at least one or plurality of processing zones are identified. The implant or rod 14 in the example is then adapted, modified or machined (block 104 in Fig. 4 and station 206 in Fig. 7A) in response to the at least one or plurality of processing zones. In this illustration for the rod 14, the rod 14 is provided with or machined to comprise generally opposing surfaces (such as generally opposing planar surfaces or areas 14a and 14b in Fig. 12) that are associated with zones 124 and 126, respectively. Recall that these zones were determined to be one or more areas of high stress during use. These areas are adapted, modified or designed (block 104 in Fig. 4, station 206 in Fig. 7A) to incorporate one or more flattened or generally planar areas to optimize peening and compression. It should be understood that the at least one processing zone or predetermined zone of the implant, such as zones 124 and 126 in the example are designed (block 102 in Fig. 4) and adapted at stations 204 and 206 to optimize compression processing (e.g., ultrasonic peening, laser peening, burnishing or chemical processing). Returning to the illustration, the surfaces 14a and 14b are the areas or regions where compression is likely to occur during use. These surfaces or areas 14a and 14b are adapted to provide, for example, clear exposure to the at least one or a plurality of compressors or peeners, such as the external laser beams from lasers 128, 130, ultrasonic peening or compression processing can occur.

Although one or more of the stations in the system 200 and steps in the method are shown or described as being separate for ease of description, it should be appreciated that a plurality or all of them could be performed at one location or station as desired. For example, designing and manufacturing may occur at one location or station, while compression, finishing and sterilization (described later) may be performed at remote locations or stations.

Thus, it should be understood that the system 200, which does not form a part of the present invention, and routine proceed to the design processing station 204 wherein the implant, such as the rod 14, is processed to implement or adapt the rod 14 at the at least one predetermined zone or processing zone to a desired shape or configuration in order to facilitate or substantially optimize the compression of the at least one predetermined zone or processing zone such as areas 124 (Fig. 7C) and 12b in rod 14 in the illustration. Note in the illustration being described, that the at least one predetermined zone or processing zone may be a shank 16b (Fig. 10) of the polyaxial screw 16, areas 12a of a plate 12, or one or more areas 124, 126 of the rod 14.

in one example, the at least one predetermined zone or processing zone oftentimes is associated with, comprises or defines at least one area that is generally equidistant between two fixation points or points where the implant, such as the rod 14, will be fixed or mounted onto a skeletal structure. It has been found that this equidistant area can be an area of high stress in the implant after the implant is mounted onto the skeletal structure in the patient. This is described in more detail later herein.

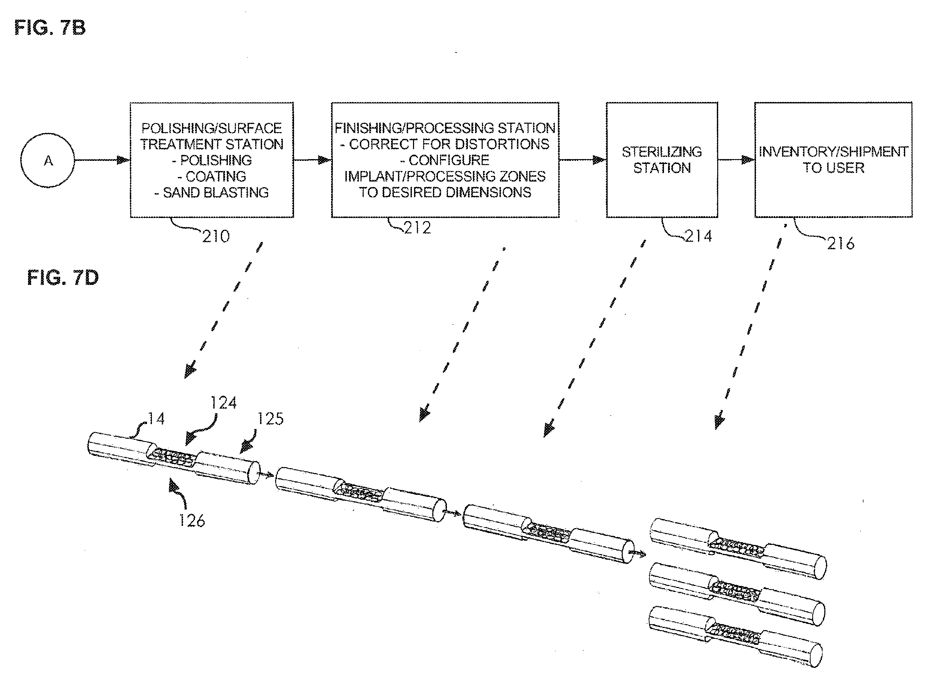

At station 206 (Fig, 7A) and at step 104 (Fig. 4) in the process, the orthopedic implant, which is the rod 14 in the example is machined, adapted, modified or manufactured at the at least one or a plurality of processing zones or areas, such as the areas 124 (Fig. 7C) and 126, using medical grade metal, such as a commercially pure titanium or extra low interstitial titanium alloy. At station 206, the rod 14 is adapted, modified and processed by machining, electrical discharge machining, molding or sintering to comprise flattened or generally planar areas, surfaces or zones as shown by surfaces 14a and 14b shown in Figs. 7C and 12. In the illustration being described, the aforementioned predetermined design adapts the rod 14 with generally flat or generally planar areas 14a, 14b which facilitates optimizing the subsequent compressing of these areas as described herein. The thickness of the rectangular cross section and any fillet radius, such as radius R associated with fillet F (Figs. 9 and 10), are engineered to balance the strength and the flexibility. In one example, it is desirable to Increase the fatigue strength of the thinner sections as high as possible. Fig. 7 of the illustration shows three unprocessed rods and manufactured rods 14 that adapted and modified and are ready for compression processing. Notice that the rods 14 have been machined to comprise machined recessed areas or notched-out areas 124a (Fig. 7C) and 126a in the illustration. It is at these areas that the rod 14 will be compressively stress processed, such as by peening. Notice that after machining, the rod 14 has generally flat or planar surfaces 14a and 14b (Fig. 11) that are receptive to and optimize compression, such as by laser shock peening.

The routine proceeds to step 106 (Fig. 4) and the system 200 (Fig. 7A) not belonging to the present invention includes a compression stress processing station 208 at which at least part or all of the at least one predetermined zone or processing zone(s) are subject to compression stress processing by one or more of the processes described earlier herein, such as by burnishing, laser shock peening (LSP), ultrasonic peening or the like.

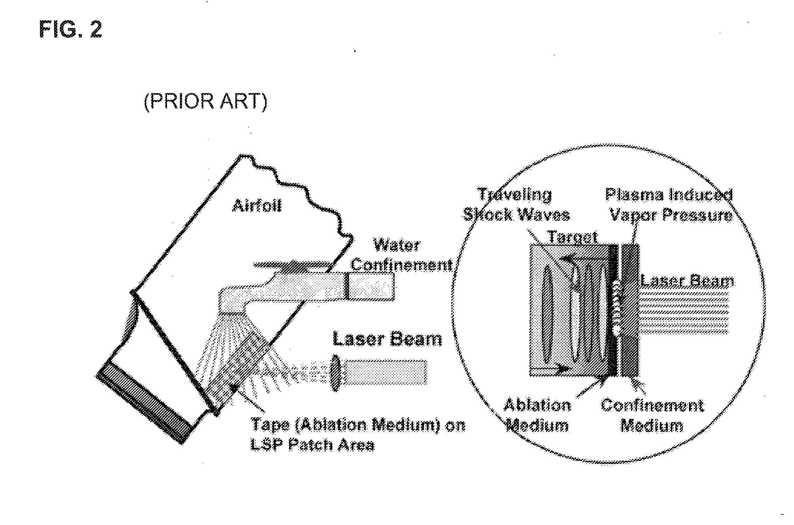

The inventors have found that it is possible to Increase fatigue strength of any component or implant if deep compressive stresses can be engineered to negate the tensile stresses experienced due to abnormal service loads. There are several methods and processes available to impart deep compressive stresses. Laser shock peening (LSP), ultrasonic peening and roller burnishing are some of these methods. Laser shock peening is a novel technology that was developed recently and is being used in aerospace industry to increase fatigue strength of aircraft engine fan and compressor blades. All three major aircraft engine manufacturers are using this technology to enhance the fatigue life and reliability of titanium alloy fan and compressor blades, the same alloy that is used as rods and pedicle screws in spinal implants. Fig. 2 shows an example of one LSP process that was used on airfoils in the prior art. Engineered application of one or more these new technologies will increase the fatigue strength of the rods. The preferred embodiment is the use of LSP process to improve fatigue strength of the rod. The process and system is engineered so that the compensating residual tensile stresses are directed to safe areas on the implant, such as rod 14, and no internal cracks are created due to the process.

In the illustration being described, the compressor used at the compression station 206 will comprise lasers 128 (Fig. 7C) and 130, which are suitable for laser shock peening. The lasers 128 and 130 are fixed and will be focused on the rod 14 and peen the rod 14 at the one or more predetermined areas or zones 124, 126 and associated surfaces 14a and 14b, respectively, on the rod 14 as the robot 201 moves the implant under one or more of the laser(s) 128 (Fig. 7C) and 130. The laser energy, pulse width and the spot size is determined from models and simulations. The spot pattern, number of hits and post processing will be determined from initial experiments. Any post processing will be determined from initial experiments. One or more of the LSP techniques, systems and methods shown in U.S. Patents 5,127,019; 5,741,559; 5,911,891; 5,911,890; 5,935,464; 5,988,982; 6,002,102; 6,049,058; 6,057,003; 6,064,035; 6,078,022; 6,127,649; 6,144,012; 6,191,385; 6,203,633; 6,236,016; 6,238,187; 6,254,703; 6,259,055; 6,288,358; 6,292,584; 6,291,794; 6,359,257; 6,373,876; 6,384,368; 6,407,375; 6,412,331; 6,462,308; 6,469,275; 6,474,135; 6,483,578; 6,483,076; 6,486,434; 6,512,584; 6,521,860; 6,528,763; 6,539,773; 6,548,782; 6,554,921; 6,566,629; 6,583,384; 6,664,506; 6,683,976; 6,747,240; 6,752,593; 6,756,104; 6,759,626; 6,841,755; 6,852,179; 6,867,390; 6,875,953; and 7,268,317 and the non-patent reference entitled Laser Shock Peening Performance and Process Simulation, by K. Ding and L. Ye, published 2006 by Woodhead Publishing Limited (Woodhead Publishing ISBN-13: 978-1-85573-929-1) may be used.

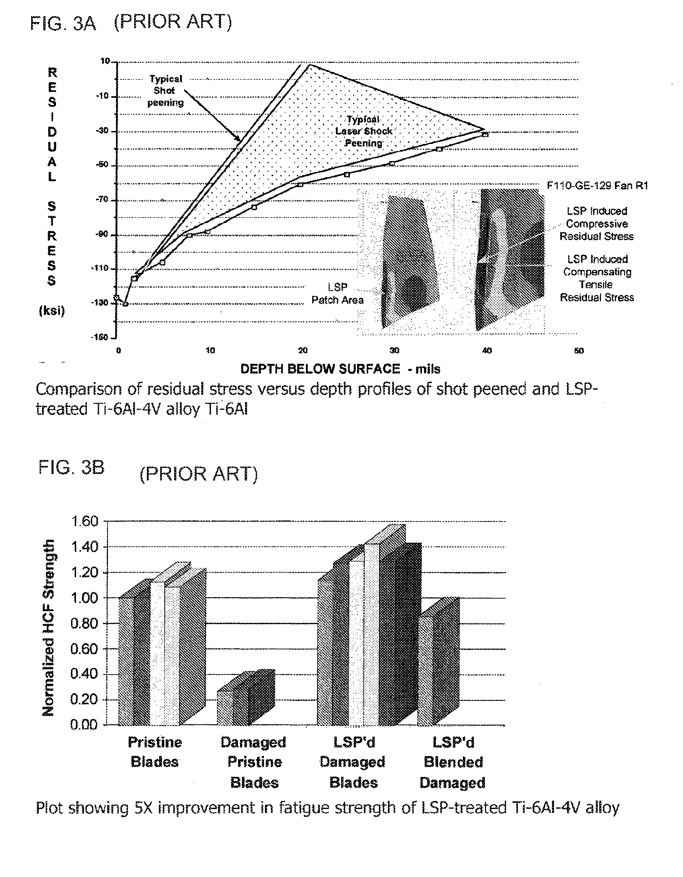

The LSP process generates deep compressive residual stresses in airfoils (Fig. 3A) through shockwaves and thereby leads to approximately five times improvement in the fatigue strength, life and resistance to crack propagation in the airfoil materials (Fig. 3B) and parts by a factor of three to five times over that provided by conventional peening treatments. As mentioned, the LSP technology is used extensively in the aerospace industry in the manufacturing of high value components. Recent findings by the inventors have indicated that LSP can significantly enhance the fatigue properties of titanium alloy stabilizing spinal rods used in implants.

Another compressive technique is burnishing. Burnishing is the plastic deformation of a surface due to sliding contact with another object. Burnishing is a process by which a smooth hard tool (using sufficient pressure) is rubbed on the metal surface. This process flattens the high spots by causing plastic flow of the metal. There are several forms of burnishing processes; the most common are roller burnishing and ball burnishing (ballizing). In both cases, a burnishing tool rubs against the work piece and plastically deforms its surface. The work piece may be at ambient temperature, or heated to reduce the forces and wear on the tool. The tool is usually hardened and coated with special materials to increase its life.

Roller Burnishing improves the finish and size of surfaces of revolution such as cylinders and conical surfaces. Both internal and external surfaces can be burnished using an appropriate tool. The plastic deformation associated with burnishing will harden the surface and generate compressive residual stresses. The benefits of burnishing often include: Combats fatigue failure, prevents corrosion and stress corrosion, textures surfaces to eliminate visual defects, closes porosity, creates surface compressive residual stress.

Low plasticity burnishing (LPB) is a method of metal improvement that provides deep, stable surface compressive residual stresses with little cold work for improved damage tolerance and metal fatigue life extension. Improved fretting fatigue and stress corrosion performance has been documented, even at elevated temperatures where the compression from other metal improvement processes relaxes. The resulting deep layer of compressive residual stress has also been shown to improve high cycle fatigue (HCF) and low cycle fatigue (LCF) performance.

Ultrasonic peening technology is another compressive technique that utilizes intense levels of high frequency acoustic energy, or high power ultrasonics, have found practical use in numerous industrial processes, of which cleaning, welding and non-destructive testing are well-known examples. Other applications include metal forming, treatment of casting materials, chemical processing, and even therapeutic and surgical uses in medicine. One of the most recent and advantageous use of high power ultrasonics in industrial applications is ultrasonic peening of metals and welded elements.

Returning to the illustration, at block 106 (Fig. 4) and station 208 (Fig. 7A), the routine proceeds to processing the orthopedic implant by providing compressive stress processing (such as by lasering or laser peening, ultrasonic or ultrasonic peening, burnishing, chemical or other compression technique) of the orthopedic implant or rod 14 at the predetermined or processing zones 124, 126 (Fig. 7C) at the compression stress processing station 208 (Fig. 7A). In one illustrative embodiment, the compression stress processing station 208 comprises the at least one or a plurality of lasers 128 and 130 (Fig. 7C) for laser peening as described later herein. As mentioned earlier, for ease of illustration, the preferred embodiment using LSP processing will be described, but it should be understood that other types of processing mentioned above could apply.

At the compression stress processing station 208, compressive stress processing of a first portion of the implant (such as the surfaces 14a, 14b of rod 14 associated with the predetermined zones or processing zones 124, 126, respectively) occurs. The surfaces 14a, 14b and associated predetermined zones 124, 126 will comprise or be adapted to comprise a tape TP or ablation medium applied thereto prior to being laser peened. As mentioned earlier, the surfaces 14a, 14b and associated predetermined zones 124, 126 or only the area(s) desired to be processed may have the biocompatible tape TP or ablation medium applied thereto, or alternatively, it may be easier to treat or provide the entire implant with the tape TP or ablation medium. In the illustration, the ablations medium is shown as the tape TP, but other mediums or coatings could be used such as, for example, a black paint or aluminum coating. During laser peening, the confinement medium mentioned earlier herein cooperates with the tape TP or ablation medium to create plasma-induced vapor pressure that creates shock waves that resonate or travel through the implant at the areas where it is laser peened. In the illustration, the confinement medium is water. As described later herein, the portions of the tape TP or ablation medium that remain on the implant after the laser peening process is complete will be removed from the implant.

After compression, the first portion or areas 124, 126 comprises a biomechanical stress concentration or first density that is generally higher than a biomechanical stress concentration or second density associated with a second portion or area, such as area 125 (Fig. 7C) of the implant body. The area 125, which is typically associated with an area that is either less likely to or is not subject to biomechanical forces after being situated on the skeletal structure in a patient. The second portion or area, such as area 125 in the example, may be either other areas of the implant that have not been treated at the compression stress processing station 208 at all or they could be other areas that are treated at the compression stress processing station 208 to have a lower density or lower biomechanical stress concentration when compared to the first portion or areas 124 and 126.

In the example, the stress processing is provided by LSP at compression stress processing station 208 which utilizes the at least one or a plurality of lasers 128, 130 to laser peen the surfaces 14a, 14b at least one predetermined zone or processing zone 124, 126 in a predetermined pattern. The lasers 128, 130 are coupled to and are under the control of the controller 132, which controls the pulse width, laser energy or laser spot size of the at least one or plurality of lasers 128 and 130. The predetermined pattern could be a continuous compression pattern, such as the pattern 134 (Fig. 13A) shown on a surface 14a' of a rod 14' described later herein relative to another embodiment. In that embodiment, the pattern 134 is continuous and extends along a length L1 parallel to a longitudinal axis of the rod 14'. In contrast and as illustrated in Fig. 8 relative to the plate 12, a predetermined pattern 136 of peening may be interrupted, such as interrupted along a length L2 or width of the plate 12 as shown. Notice in Fig. 8 that the predetermined pattern is not continuous along the entire length L2 of the plate 12.

Returning to the illustration regarding the rod 14, the one or more laser beams from lasers 128, 130 are applied to the surfaces 14a, 14b (Fig. 12) associated with the processing zones 124 and 126, respectively, in the desired or predetermined pattern 138 (Fig. 11). In one illustrative embodiment, the at least one or plurality of lasers 128, 130 may have an energy output in the range of 10-500J/pulse with a pulse duration of less than about 100ns and a laser spot size of less than about 20 mm. In this embodiment, the pattern 138 is an overlapping pattern as shown in Fig. 11. This overlapping pattern is created by the robot 201 (Fig. C) passing the rod 14 back and forth under the lasers 128 and 130 or, alternatively, causing laser beams from lasers 128 and 130 to make multiple passes over each of the surfaces 14a and 14b. It has been found that the overlapping pattern provides more compressive loading, compared to non-overlapping patterns, at particular areas in the processing zones and improves the density of the part, such the rod 14, at the processing zones associated with areas 124 and 126.

As illustrated in the various examples in Figs. 8-16, note that the predetermined pattern of peening may be rectangular, circular, elliptical, polyaxial, linear, helical, spiral or even overlapping (Fig. 11). Figs. 15 and 16 illustrate application of a helical or spiral pattern on a generally cylindrical or continuous radius surface 14a'" of a rod 14"' in another example described later herein. As mentioned earlier, Figs. 13A and 14A illustrate a generally continuous elongated compression pattern on different implants.

The processing zones could be either partially or entirely processed or subject to the compressive stress processing, such as by using the lasers 128 and 130. Note also that one surface, such as surface 14a of rod 14 (Fig. 7C), may be processed at a time, or multiple surfaces, such as surfaces 14a and 14b, may be processed concurrently.

In general, the entire or substantially all of the first portion or area associated with predetermined zone or processing zone, such as surfaces 14a and 14b for rod 14, will be treated or processed with the compressive stress processing, but it should be understood that less than all of the surface 14a may be treated or only particular areas of the implant may be treated. In a preferred embodiment, it is desired to treat the areas or predetermined zones of the implant that will experience high stress during use and not to treat other areas, such as second portion or area 125 (Fig. 7C), of the implant. Thus, the compression processing could be applied to the entire area defined by the surfaces 14a, 14b associated with the predetermined zone or processing zone 124, 126, respectively. Alternatively, only a portion of the predetermined zone or processing zone may be processed. For example, in the illustration shown in Fig. 8, the plate 12 may be compressive stress treated at the areas, such areas 12a between aperture pairs, such as pairs 140, 142 and 144, 146 or areas 12c and 12d around apertures 140 and 142, respectively. As mentioned earlier, plate 12 in Fig. 8 may be processed or peened at compression stress processing station 208 so that the peened predetermined zones or processing zones are interrupted, for example, along the longitudinal length L2 of the implant.

Continuing with the illustrative system 200 (Figs. 7A - 7B) and procedure (Figs. 4 - 6), after the compression step (block 106) and compression stress processing station 208, the implant, such as the rod 14, is processed (Block 108 in Fig. 4) at the polishing surface treatment station 210 (Fig. 7B). At station 210, the implant is polished or surface treated. In this regard, such polishing or surface treatment may include electropolishing, mechanical polishing, coating, media blasting (e.g., sand blasting) or machining.

During the processing step in block 106 and at the compression stress processing station 208, the rod 14 may become disfigured or outside of desired dimensional tolerances. For example, the width W (Fig. 12) of the rod 14 at the processing zone or area 124 could become greater than desired after the compressive stressing step in block 106 that occurs at the compression stress processing station 208 or the entire cross-section could become bowed as a result of compression. Accordingly, the system 200 may include the finish processing station 212 (Fig. 7B) and the process may include the step of processing or treating the implant (block 110 in Fig. 4) to correct such tolerance, dimensional or configuration issues, such as to correct dimensional intolerances or to configure the implant to a desired shape or dimension. Such additional processing may include, for example, lathe cutting, milling, drilling, grinding, forming or other conventional machining steps. At process blocks 110 (Fig. 4) and blocks 110a (Fig. 6) and 110b and at a finish processing station 212, the process and system 200 continues and the implant, such as rod 14, is inspected or checked for configuration or tolerance concerns, such as distortion modifications (block 110a in Fig. 6). If further processing is required, then the implant body or rod 14 in the illustration is machined or processed further to reduce or adjust the Implant for the surface distortions, modifications or to perform any further processing that may be required.

After the processing block or step 110 (Fig. 4) at finish processing station 212 (Fig. 7B), the implant body is polished again or for the first time before sterilization at a sterilizing station 214. During this step 112 and at sterilizing station 214, the implant or rod 14 in the illustration is subject to sterilization utilizing gamma irradiation, steam, heat, chemically, such as by use of ethylene oxide, or other modality to reduce implant bioburden. It should be understood that any biocompatible tape TP (Fig. 7C) or ablation medium that remains after the implant is laser peened is removed prior to or during the sterilization process, or it could be removed separately during a biocompatible ablation tape or layer removal process. As mentioned earlier, the sterilization may occur at a location that is physically remote from the stations 202 - 212.

At station 216 (Fig. 7B), the finished and sterilized implants are stored in inventory and/or shipped to a user. Figs. 7B - 7C show the progression of the rod 14 as the system and method are applied. Accordingly, the process may include the step 114 of the surgeon implanting the processed orthopedic implant 10 onto the skeletal structure (not shown) of the patient.

Advantageously, the system 200 and method described earlier relative to Figs. 4-7C and which both do not constitute a part of the present invention provide a system and method for modifying, processing or making an implant having the at least one first portion or area, such as area 124 and/or area 126, that is compressed so that the density at the area is higher than a density of the second portion or area, such as area 125 of the implant, thereby providing an implant with the first portion or area that comprises a biomechanical stress concentration or density that is higher than a biomechanical stress concentration or density of the second portion, particularly when the implant is subject to biomechanical forces after being situated on a skeletal structure in a patient. The first portion of the implant body, that is, the portion that has a higher biomechanical stress concentration or density than the second portion or area, may be an area that has been compressively stressed as described herein, while the second portion may comprise an area, such as area 125 (Fig. 7C), that is not compressively stressed at all or that has been compressively stressed, but to a lesser degree or density.

As mentioned earlier, the LSP process described herein imparts the compressive stresses in at least one predetermined zone or processing zone or in a plurality of zones as described herein. It should be understood that the compensating tensile stresses experienced during use will be generated or transmitted outside the at least one predetermined zone or processing zone, such as to the area 125 (Fig. 7C). Again, it has been found that the optimal way to protect the implant from failure is to impart the compressive stresses through the entire thickness of the area of interest, such as the at least one predetermined zone or processing zone, to assure that the rod 14 will not fail due to bending tensile stresses.

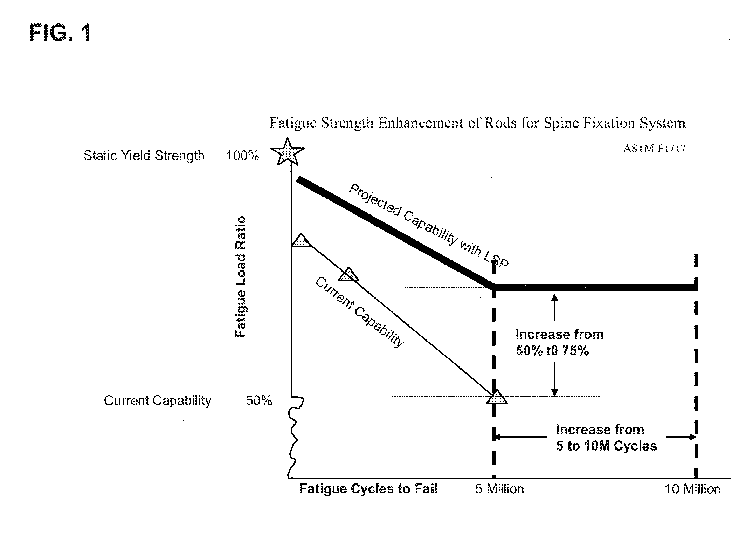

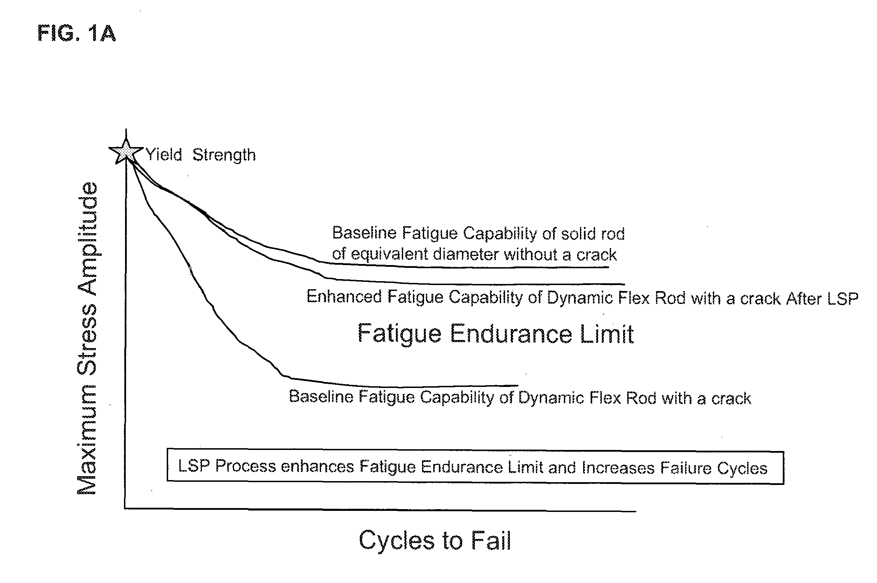

Figs. 1 and 1A illustrate the fatigue strength enforcement of rods in the illustration being described. These Figs. 1 and 1A illustrate that the LSP process enhances fatigue endurance limit and increases the fatigue capability of the implant. Note in the graph in Figs. 1 and, 1A the comparison of various characteristics of a rod (not shown) with a crack, a rod with a crack (not shown) after LSP, and then a rod, such as rod 14, without a crack after LSP. Note the fatigue endurance limit for those rods 14 after LSP is increased. This means that the LSP treated implant enhances fatigue endurance limit and increases failure cycles.

As described herein, the system and method not belonging to the present invention provide means for modifying, processing or making an orthopedic implant, such as the implants shown in Figs. 9-16, but it should be understood that it is within the scope of the invention that the system and method be used to make other types of implants as well. As mentioned earlier, the system is particularly usefol in making orthopedic implant plates 12 (Fig. 8), rods 14 (Figs. 9, 11, 12 and 13A - 16) and polyaxial screws 16 (Fig. 10).

As illustrated in Fig. 8 and as alluded to earlier, the high biomechanical stress areas are the areas between fixation points, such as areas 12a in the plate 12 in Fig. 8. The high biomechanical stress areas are oftentimes found to be generally equidistant between fixation points where the implant is fixed to the skeletal structure. These are often areas of high torque during the patient's movement after the implant has been implanted in the patient. For the plate 12 (Fig. 8), these areas correspond to the areas where screws secure the plate 12 to skeletal bone. For example, the aperture pairs 140 (Fig. 8), 142 and apertures 144 and 146 In plate 12 each receive a screw (not shown). The area 12a1 between these pairs of apertures 140, 142 and 144, 146 experiences high stress during use. Consequently, this area 12a1 is a predetermined zone or processing zone that receives compressive stress treatment. In this example, note that the plate 12 is processed in accordance with the method and system described herein but not forming part of the present invention so that these areas 12a 1 and 12a2 are generally recessed and facilitate optimizing compression during the LSP process.

Note that in the case of the polyaxial screw 16 (Fig. 10), the biomechanical stress concentration is oftentimes highest at the shank area 16b between a screw head 16a and screw threads 16c. In the example, this predetermined zone or processing zone is designed, processed and machined at stations 202 - 206 to have generally planar surfaces 16d and 16e to optimize LSP. The LSP processing strengthens and densifies the implant at this area 16b. As with the rod 14 and plate 12, therefore, the at least one predetermined zone or processing zone is associated with surfaces 16d and 16e, which become generally planar or flattened to optimize the laser peening at the compression stress processing station 208 as described earlier herein.

Thus, it should be appreciated that the geometry of the implant is extremely important for effective use of the LSP process and compressive stress processing. It has been found that it is difficult to accomplish, through thickening the implant, compressive stresses for circular cross-section rods. Figs. 13A-13C illustrates various examples where the circular cross-section of the rod 14' has been modified to optimize the LSP process. In Fig. 13B, the rod 14" has been modified with the single generally flattened or planar surface 14a' along Its longitudinal length L1. Fig. 13C illustrates a plurality of surfaces, surfaces 14a' and 14c', which are generally opposed and again facilitate peening, such as by LSP. If the LSP process can produce through thickness compensation up to 1 mm depth D1 (Fig. 13B), it can be seen from Figs. 13A-13C that the modified cross-section can have deeper protection than the circular cross-section. With thinner cross-sectional widths, larger volumes of area can be treated and will result in deeper LSP treatment.

In the example shown in Figs. 13A - 13B, the rod 14' is redesigned at the design stations 202 and 204 with overall dimensions of approximately 4mm and with flat sections at the surface 14a'. It should also be understood that another surface 14c' on the rod 14' as is shown in Fig. 13C. As with prior examples, the rod 14' is redesigned with at least one generally flattened or planar surface, such as at the surfaces 14a' and 14c', to optimize the LSP process.

Figs. 14A-14C illustrate an embodiment of the Invention. In this embodiment, a rod 14" is machined or adapted at station 206 (Fig. 7A) to comprise at least one or a plurality of the peripherally-shaped lobes 14h"-14k" (Fig. 14A) that extend longitudinally along a length L3 of the rod 14". The rod 14" is machined from a cylindrical rod (not shown). One or more of the lobes 14h"-14k" are designed, adapted and/or machined at stations 202 - 206 to comprise at least one or a plurality of predetermined zones or processing zones that have been optimized to receive the compressive stress processing described herein. In the embodiment being described, note that the generally planar or flattened surface 14i1" (Fig. 14A) has been adapted and made generally planar to optimize the peening process, such as LSP as described herein, at the vortex of the lobe 14i".

Optionally, the opposing surface 14k" may also be processed similarly so that the opposing surfaces 14i1" and 14k1" have been compressively stress treated by, for example, the LSP process described earlier herein relative to the compression stress processing station 208. It should be understood that during use, it is intended that the rod 14" will be placed on the skeletal structure such that it bends along the longitudinal axis so that one of the at least one predetermined zone or processing zone, such as the zone associated with the surface 14i1", may be under tension, while the opposing surface 14k1" is under compression or vice versa.

As mentioned earlier, the predetermined pattern of peening at least one predetermined zone or processing zone may be interrupted along a longitudinal axis of the implant, such as is shown and described earlier relative to the plate 12 in Fig. 8, or it could extend along only a portion of the implant, as is illustrated relative to the rod 14 in Figs. 9, 11 and 12 and the screw 16 shown in Fig. 10. Alternatively, and as is illustrated in Figs. 13A-16, the compressive stress areas may extend along the entire length of the implant as is shown in the rods 14' (Fig. 13A) and 14" (Fig. 14A) and 14"' (Fig. 16).

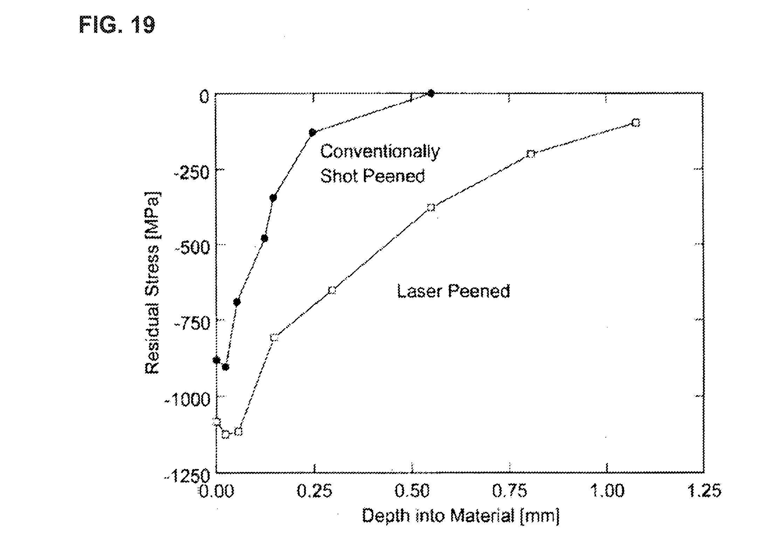

Returning back to the illustration and embodiment shown in Figs. 14A and 14B, note that the thickness T1 of the lobes 14h" and 14j" is substantially larger than the thickness T2 of the lobes 14i" and 14k". As alluded to earlier, the LSP process can produce compression up to about 1 mm in depth D2 (Fig. 14B) and it has been found that this thickness is larger than the laser peening compression depth D1 (Fig. 13B) associated with the rod 14'. In other words, the design or shape of the rod 14" and the various lobes 14h"-14k" permit a deeper compressive stress processing at the tip or vertex of the one or more of the lobes, such as lobes 14i" and 14k" when they are laser peened. Fig. 19 illustrates the improved residual stress versus depth characteristics for an implant, such as rod 14", that has been laser shock peened and a theoretical comparison to an implant (not shown) that was conventionally peened. Note that a depth associated with an increase in the residual stress increases with the laser-peened implant and this can be controlled or enhanced depending on the design of the implant. For example, Figs. 14A and 14B show the lobes 14h"-14j" having larger widths than lobes 14i" and 14k". The thinner widths allow deeper penetration of the LSP and improved residual stress to depths in the surface of the implant that were greater than what could be achieved in the past with traditional LSP.

Notice also, that the rod 14" has less material than the rod 14 or 14' and less implant volume when compared to a regular rod, but yet can comprise greater tensile stresses during use when compared to a regular, non-treated rod.

As illustrated in Figs. 14A and 14B, a first pair of the peripherally-shaped lobes, such as lobes 14h" and 14j", lie in a first plane and lobes 14i and 14k lie in a second plane that is generally perpendicular to the first plane. It should be understood, however, that the lobes 14h"-14k" may be provided in other planes and in other orientations as may be desired or determined at the design stations 202, 204.

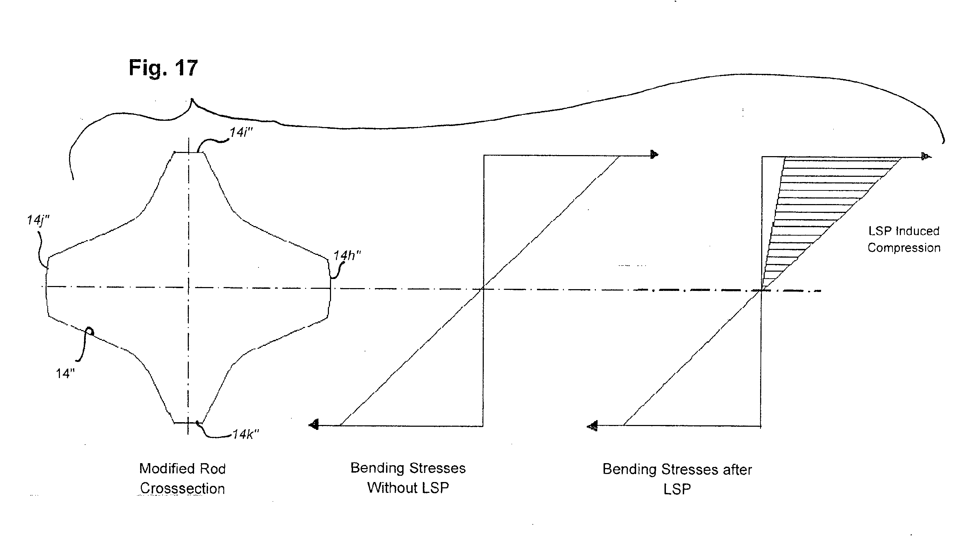

Stress profiles due to rod 14" bending for the embodiment of Figs. 14A - 14B are illustrated in Fig. 17. The top section or surface 14i1" experiences tensile stresses, for example, while the bottom section or surface 14k1" experiences compressive stresses, with zero stresses in the neutral axis, which is the axis between lobes 14h" and 14j". Advantageously, the effect of the compressive stress processing, such as by peening using LSP, at the surfaces 14i1" and 14k1" introduce compressive stresses to negate the bending induced tensile stresses, thereby increasing the fatigue-handling capabilities of the rod 14".

Thus, it should be understood that at least one or a plurality of the peripherally-shaped lobes 14h"-14k" may be densified by LSP to provide the second portion or area which defines the area at which the compressive stress processing or laser peening occurs, such as at the surface 14i1" in the illustration in Fig. 14A.

Note that each of the peripherally-shaped lobes 14h"-14k" comprises generally tapered surfaces, such as surfaces 14h2" (Fig. 14B) and 14i2". The generally tapered surfaces, such as surfaces 14h2" and 14i2", are adapted-to interconnect with mating surfaces of another implant component, such as a tulip receiver, pedicle screw or compression member. Accordingly, one feature of the embodiment being described is that the implant may be designed, adapted and machined at stations 202, 204 and 206 to comprise surfaces that not only have the predetermined zones or processing zones and associated shapes that are adapted for optimizing the laser shock peening, but also are designed and adapted to accommodate and interconnect with at least one other implant component. Returning to the embodiment being described, it should be appreciated that after processing at station 208 and steps 100 - 106 in the process as provided herein, the density at the tips or surfaces 14i1" and 14k1" is greater than the density of the material at the surfaces 14h1" and 14j1".

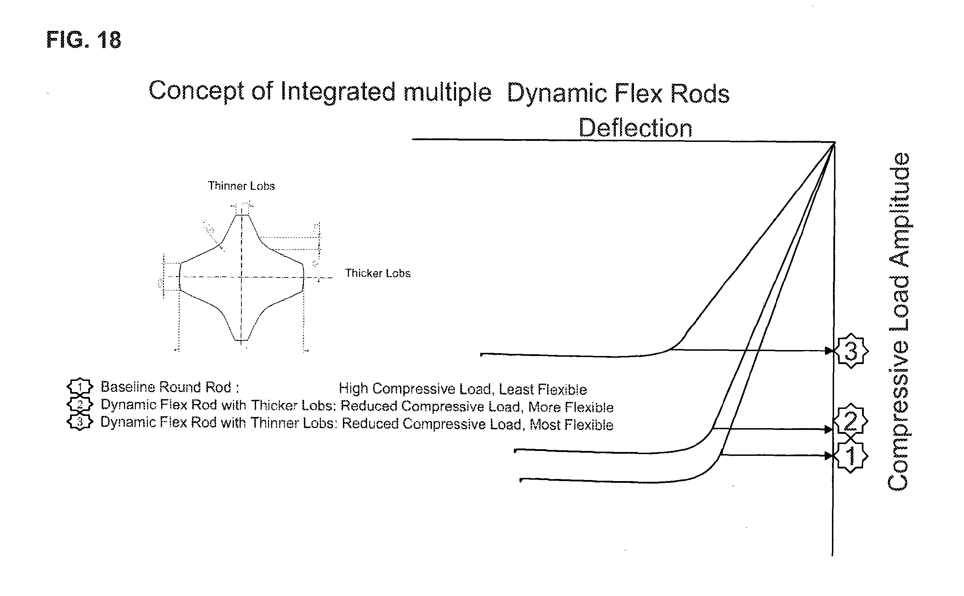

One feature of the embodiment of Fig. 14A is that the rod 14" shape is designed to not only enhance LSP, but also to provide the rod 14" with multiple dynamic compressive load and flexibility characteristics. Fig. 18 illustrates this concept. Note that a baseline rod (not shown), labeled 1 in the graph, has a baseline high compressive load and flexibility characteristic. The rod 14" thicker lobes 14h" and 14j"(Fig. 14B) comprises the compressive load amplitude and flexibility that is greater than the baseline rod, as indicated by the comparison of lines 1 and 2 in the graph in Fig. 18. The thinner lobes 14i" and 14k" provide the most flexibility as illustrated by the line 3 in the graph of Fig. 18.

Advantageously, the implant described herein can be designed and provided with multiple dynamic compression and flexion characteristics as illustrated. In the illustration of Fig, 14A-14B, this means that the rod 14" was designed with uniquely shaped and sized lobes so that its compression and flexion characteristics along its length in the example were different depending on the orientation of the rod 14" and the direction of flexation. This provides greater application and flexibility of use of the rod 14". For example, the surgeon may orient and mount the rod 14" on a patient so that lobes 14i" and 14k" (Fig. 14B) are under compression and tension, respectively, which provides reduced compressive load (compared to when the rod 14" is oriented so that lobes 14h" and 14j" are under compression/tension), but more flexibility for the patient, as illustrated in Fig, 18. In contrast, the surgeon may orient the rod 14" so that the lobes 14h" and 14J" are under compression and tension, respectively, which means the rod 14" will have lower compressive load characteristics, compared to the thinner lobes 14i" and 14k", but less flexibility. Thus, the rod 14" will be oriented and mounted depending on the compressive load and flexibility requirements. One advantage of the embodiment of Figs. 14A and 14B is that it provides an implant having multiple compression and flexion characteristics, which enables multiple uses of the rod 14".

While the illustration shown and described herein shows two pairs of lobes, it should be understood that more or fewer lobes or pairs of lobes may be provided so that the implant can have one or a plurality of compressive load and flexibility characteristics to enhance the uses of the implant or increase the number of applications in which it may be used. Thus, in the embodiment, an implant is provided that comprises a plurality of dynamic flexion and load characteristics that are defined by a plurality of pairs of generally opposing surfaces, such as the pairs of lobes 14h"-14j" and 14i"-14k" that were treated by LSP.

Again, it should be appreciated that the implant is designed to overcome or negate bending-induced tensile stresses. This can occur at areas of the implant or surface of the implant where it interfaces skeletal bone. The bone interface at which the implant contacts the skeletal bone can define one or more areas of highest stress during use after the implant body is mounted onto a skeletal structure. For example and as mentioned earlier, it could be the areas around or between screw aperture pairs 140, 142 and 144, 146 in plate 12, which does however not constitute a part of the present invention. As mentioned earlier, the area 16b of the screw 16 in Fig. 10 that extends outside the bone tends to be the area of highest fatigue and stress during use after the implant body is mounted onto the skeletal structure. Accordingly, in the illustration being described, the generally planar sides 16d and 16e are compressive stress processed as shown relative to Figs. 10 and 10A.

Various figures show different predetermined designs and patterns which utilize generally planar or generally flattened areas that have been optimized to receive compression, such as by LSP, as described herein. In the previous illustrations, the implants have been processed by LSP, along a length or at least a portion of a length or a width of the implant, with the peening being interrupted, such as interrupted along the length as shown in non-inventive plate 12 in Fig. 8, or uninterrupted as illustrated with the inventive rods 14' and 14" (Figs. 13A and 14A, respectively). Figs. 15 and 16 illustrate another example of treating an implant that is cylindrical or that comprises an outer radius.

Figs. 15 and 16 illustrate another example not forming a part of the present invention for treating a continuous outer radius of an implant, such as a rod 14"' with laser shock peening. In this example, the robot 201 having the holder 201a holds the part or rod 14"' and causes relative movement axially and rotationally as the lasers 128 and 130 laser shock peen the outer circumference or radial surface 14a"' of the rod 14"', The controller 132 controls the lasers 128 and 130 to laser peen the desired pattern on the surface 14a"' during movement of the rod 14"'. As with prior examples not forming part of the present invention, the field frequency, size, pattern of overlap, pulse width and the like may be changed to obtain the desired laser shock peening treatment result. This movement of the implant during the LSP process achieves a spiral or helical pattern as illustrated in Fig. 15 that maximizes LSP coverage. The spiral or helical treatment geometry results in maximal surface treatment of the rod 14"' utilizing at least one or a plurality of laser fields from lasers 128 and 130.

While the Implants shown an described herein constitute preferred embodiments of this invention, it is to be understood that the invention is not limited to this preciseimplant, and that changes may be made without departing from the scope of the invention, which is defined in the appended claims.

## Claims

1. An orthopedic implant (14) comprising:
an implant body;
a first portion of said implant body have a first density; and
a second portion of said implant body has a second density;
wherein said first portion is compressed so that said first portion comprises a biomechanical stress concentration or density that is higher than a biomechanical stress concentration or density in said second portion when said orthopedic implant (14) is subject to biomechanical forces after being situated on a skeletal structure, said implant body being elongated, **characterized in that** said implant body comprises a plurality of peripherally-spaced lobes (14h", 14i", 14j", 14k"), at least one of said plurality of peripherally-spaced lobes (14h", 14i", 14j", 14k") extending longitudinally along the entire length of said implant body and adapted to provide said first portion.

2. The orthopedic implant (14) as recited in claim 1, **characterized in that** selective ones of said plurality of peripherally-spaced lobes (14h", 14i", 14j", 14k") that comprise said first portion also comprise a thickness (T2) in cross section that is less than a second thickness (T1) of said at least one other (14h", 14j") of said plurality of peripherally-spaced lobes (14h", 14i", 14j", 14k") that comprise said second portion.

3. The orthopedic implant (14) as recited in claim 1, **characterized in that** said at least one (14i", 14k") of said plurality of peripherally-spaced lobes (14h", 14i", 14j", 14k") is densified by laser peening to provide said first portion.

4. The orthopedic implant (14) as recited in claim 1, **characterized in that** said plurality of peripherally-spaced lobes (14h", 14i", 14j", 14k") comprise a first lobe (14i") and a generally opposing second lobe (14k"), each of said first and second lobes (14i", 14k") being densified by laser peening.

5. The orthopedic implant (14) as recited in claim 1, **characterized in that** a first pair (14i", 14k") of said plurality of peripherally-spaced lobes (14h", 14i", 14j", 14k") are generally opposed and lie in a first plane and a second pair (14h", 14j") of said plurality of peripherally-spaced lobes (14h", 14i", 14j", 14k") lie in a second plane, each of said plurality of peripherally-spaced lobes (14h", 14i", 14j", 14k") having tapered sides.

6. The orthopedic implant as recited in claim 5, **characterized in that** said first pair (14i", 14k") of said plurality of peripherally-spaced lobes (14h", 14i", 14j", 14k") are adapted to define said first portion and comprise a first lobe density and said second pair (14h", 14j") of said plurality of peripherally-spaced lobes (14h", 14i", 14j", 14k") comprise a second lobe density, said first lobe density being greater than said second lobe density.

7. The orthopaedic implant (14) as recited in any of claims 1 to 6, **characterized in that** the implant (14) is a rod (14").

8. The orthopaedic implant (14) as recited in any of claims 1 to 7, **characterized In that** the shapes and sizes of the peripherally-spaced lobes (14h", 14i", 14j", 14k") are adapted to provide specific compression and flexion characteristics to the orthopaedic implant (14) along its length.

## Patentansprüche

1. Orthopädisches Implantat (14), umfassend:
einen Implantatkörper;
einen ersten Abschnitt des Implantatkörpers mit einer ersten Dichte; und
einen zweiten Abschnitt des Implantatkörpers mit einer zweiten Dichte;
worin der erste Abschnitt komprimiert ist, so dass der erste Abschnitt eine biomechanische Spannungskonzentration oder Dichte umfasst, die höher als eine biomechanische Spannungskonzentration oder Dichte im zweiten Abschnitt ist, wenn das orthopädische Implantat (14) nach Positionierung auf einer Skelettstruktur biomechanischen Kräften ausgesetzt ist, wobei der Implantatkörper länglich ist, **dadurch gekennzeichnet, dass** der Implantatkörper eine Vielzahl von peripher beabstandeten Nocken (14h", 14i", 14j", 14k") umfasst, wobei sich zumindest einer der Vielzahl von peripher beabstandeten Nocken (14h", 14i", 14j", 14k") in Längsrichtung über die gesamte Länge des Implantatkörpers erstreckt und den ersten Abschnitt bereitstellt.

2. Orthopädisches Implantat (14) nach Anspruch 1, **dadurch gekennzeichnet, dass** ausgewählte Nocken aus der Vielzahl von peripher beabstandeten Nocken (14h", 14i", 14j", 14k"), die den ersten Abschnitt bereitstellen, eine Dicke (T2) im Querschnitt aufweisen, die kleiner ist als eine zweite Dicke (T1) von zumindest einem anderen Nocken (14h", 14j") aus der Vielzahl von peripher beabstandeten Nocken (14h", 14i", 14j", 14k"), der den zweiten Abschnitt bereitstellt.

3. Orthopädisches Implantat (14) nach Anspruch 1, **dadurch gekennzeichnet, dass** zumindest ein Nocken (14i", 14k") aus der Vielzahl von peripher beabstandeten Nocken (14h", 14i", 14j", 14k") durch Laserpeening verdichtet wird, um den ersten Abschnitt bereitzustellen.

4. Orthopädisches Implantat (14) nach Anspruch 1, **dadurch gekennzeichnet, dass** die Vielzahl von peripher beabstandeten Nocken (14h", 14i", 14j", 14k") einen ersten Nocken (14i") und einen im Wesentlichen gegenüberliegenden zweiten Nocken (14k") umfassen, wobei der erste und der zweite Nocken (14i", 14k") je durch Laserpeening verdichtet ist.

5. Orthopädisches Implantat (14) nach Anspruch 1, **dadurch gekennzeichnet, dass** sich ein erstes Paar (14i", 14k") aus der Vielzahl von peripher beabstandeten Nocken (14h", 14i", 14j", 14k") im Wesentlichen gegenüber und in einer ersten Ebene liegt, und dass ein zweites Paar (14h", 14j") aus der Vielzahl von peripher beabstandeten Nocken (14h", 14i", 14j", 14k") in einer zweiten Ebene liegt, wobei jeder aus der Vielzahl von peripher beabstandeten Nocken (14h", 14i", 14j", 14k") sich verjüngende Seiten aufweist.

6. Orthopädisches Implantat nach Anspruch 5, **dadurch gekennzeichnet, dass** das erste Paar (14i", 14k") aus der Vielzahl von peripher beabstandeten Nocken (14h", 14i", 14j", 14k") den ersten Abschnitt bereitstellt und eine erste Dichte der Nocken aufweist, und dass das zweite Paar (14h", 14j") aus der Vielzahl von peripher beabstandeten Nocken (14h", 14i", 14j", 14k") eine zweite Dichte der Nocken aufweist, wobei die erste Dichte der Nocken größer ist als die zweite Dichte der Nocken.

7. Orthopädisches Implantat (14) nach einem der Ansprüche 1 bis 6, **dadurch gekennzeichnet, dass** das Implantat (14) ein Stab (14") ist.

8. Orthopädisches Implantat (14) nach einem der Ansprüche 1 bis 7, **dadurch gekennzeichnet, dass** die Formen und Größen der peripher beabstandeten Nocken (14h", 14i", 14j", 14k") so ausgelegt sind, dass sie dem orthopädischen Implantat (14) über seine Länge bestimmte Kompressions- und Biegeeigenschaften verleihen.

## Revendications

1. Implant orthopédique (14), comprenant:
un corps d'implant;
une première partie dudit corps d'implant présente une première densité; et
une deuxième partie dudit corps d'implant présente une deuxième densité,
dans lequel ladite première partie est comprimée de telle sorte que ladite première partie présente une concentration ou une densité de contrainte biomécanique qui est supérieure à une concentration ou une densité de contrainte biomécanique dans ladite deuxième partie lorsque ledit implant orthopédique (14) est soumis à des forces biomécaniques après avoir été placé sur une structure de squelette, ledit corps d'implant étant allongé, **caractérisé en ce que** ledit corps d'implant comprend une pluralité de lobes périphériquement espacés (14h", 14i", 14j", 14k"), au moins un de ladite pluralité de lobes périphériquement espacés (14h", 14i", 14j", 14k") s'étendant de façon longitudinale le long de la totalité de la longueur dudit corps d'implant et étant adapté pour former ladite première partie.

2. Implant orthopédique (14) selon la revendication 1, **caractérisé en ce que** des lobes sélectifs de ladite pluralité de lobes périphériquement espacés (14h", 14i", 14j", 14k") qui comprennent ladite première partie présentent également une épaisseur (T2) en section transversale qui est inférieure à une deuxième épaisseur (T1) dudit au moins un autre lobe (14h", 14j") de ladite pluralité de lobes périphériquement espacés (14h", 14i", 14j", 14k") qui comprend ladite deuxième partie.

3. Implant orthopédique (14) selon la revendication 1, **caractérisé en ce que** ledit au moins un lobe (19i", 14k") de ladite pluralité de lobes périphériquement espacés (14h", 14i", 14j", 14k") est densifié par martelage au laser afin de former ladite première partie.

4. Implant orthopédique (14) selon la revendication 1, **caractérisé en ce que** ladite pluralité de lobes périphériquement espacés (14h", 14i", 14j, 19k") comprend un premier lobe (14i") et un deuxième lobe essentiellement opposé (14k"), chacun desdits premier et deuxième lobes (14i", 14k") étant densifié par martelage au laser.

5. Implant orthopédique (14) selon la revendication 1, **caractérisé en ce qu'**une première paire (14i", 14k") de ladite pluralité de lobes périphériquement espacés (14h", 14i", 14j", 14k") est généralement opposée et est située dans un premier plan, et une deuxième paire (14h", 14j") de ladite pluralité de lobes périphériquement espacés (19h", 14i", 14j", 14k") est située dans un deuxième plan, chacun de ladite pluralité de lobes périphériquement espacés (14h", 14i", 14j", 14k") présentant des côtés coniques.

6. Implant orthopédique selon la revendication 5, **caractérisé en ce que** ladite première paire (14i", 14k") de ladite pluralité de lobes périphériquement espacés (14h", 14i", 14j", 14k") est adaptée pour définir ladite première partie et présente une première densité de lobe, et ladite deuxième paire (14h", 14j") de ladite pluralité de lobes périphériquement espacés (14h", 14i", 14j", 14k") présente une deuxième densité de lobe, ladite première densité de lobe étant supérieure à ladite deuxième densité de lobe.

7. Implant orthopédique (14) selon l'une quelconque des revendications 1 à 6, **caractérisé en ce que** l'implant (14) est une tige (14").

8. Implant orthopédique (14) selon l'une quelconque des revendications 1 à 7, **caractérisé en ce que** les formes et les tailles des lobes périphériquement espacés (14h", 14i", 79j", 14k") sont adaptées pour fournir des caractéristiques de compression et de flexion spécifiques à l'implant orthopédique (14) le long de sa longueur.
